# EUROPEAN PATENT APPLICATION

(11) **EP 4 201 477 A1**
(43) Date of publication of application: **28.06.2023**
(21) Application number: 22209253.8
(22) Date of filing: 24.11.2022
(51) Int. Cl.: A61N 2/00

(54) **MUSCLE STIMULATION APPARATUS USING MAGNETIC FIELD PULSE**

(30) Priority: 22.12.2021 KR 20210185055
(71) Applicant: Lutronic Corporation, Goyang-si, Gyeonggi-do 10534 (KR)
(72) Inventor: KO, Kwang Chon, 10893 Paju-si, Gyeonggi-do (KR)
(74) Representative: Michalski Hüttermann & Partner Patentanwälte mbB

(57) **Abstract**

In the muscle stimulation apparatus using a magnetic field pulse, the method of controlling the same, and the method of stimulating a muscle using the same according to the present disclosure, it is possible to set the depth and the intensity of stimulation based on muscle mass and the thickness of a fat layer. Accordingly, it is possible to perform muscle stimulation optimized for each individual and each muscle while reducing pain and minimizing any possible inconvenience in using the apparatus.

## Description

### BACKGROUND OF INVENTION

### Field of the invention

In recent days, a method of causing muscle contraction by electrically stimulating muscles has been used for the purposes of treatment, rehabilitation, muscle strengthening, or fat reduction. When a muscle is contracted by electrical stimulation, the intensity of the muscle contraction may be determined by the frequency and intensity of a current applied from an apparatus regardless of whether a user intends or not.

The method of stimulating a muscle with electricity as described above can be classified into a method in which a current is directly flown into the muscle and a method in which an induced current is generated based on a magnetic field pulse. In connection with the method using a magnetic field pulse, Korean Patent No. 2195670 discloses a technique for stimulating the sphincter to treat urinary incontinence based on a magnetic field pulse.

However, in the case of such a prior art, there is a problem in that it is not possible to optimize the depth and intensity of stimulation for each individual.

### SUMMARY

The purpose of the present disclosure is to provide a muscle stimulation apparatus using a magnetic field pulse optimized for each individual and each muscle, a method of controlling the same, and a method of stimulating a muscle using the same by resolving the problem of the conventional muscle stimulation apparatus using a magnetic field pulse.

To resolve the problem of the conventional muscle stimulation apparatus using a magnetic field pulse, according to an aspect of the present disclosure, there is provided a muscle stimulation apparatus using a magnetic field pulse, including a power supply unit, an applicator including a magnetic field generator capable of generating a magnetic field pulse by receiving power from the power supply unit, and a controller capable of setting at least one of the frequency and the intensity of a magnetic field pulse generated by the magnetic field generator, wherein the controller adjusts a parameter for deciding at least one of the frequency and the intensity of the magnetic field pulse in the middle of performance of a preset treatment process, and a value of the parameter is determined based on at least one of the amount of muscle to be stimulated and the thickness of a fat layer outside the muscle of a user.

According to another aspect of the present disclosure, there is provided a method of controlling the muscle stimulation apparatus using a magnetic field pulse, including the step of deciding a parameter based on at least one of the amount of muscle to be stimulated and the thickness of a fat layer outside the muscle of a user, the step of deciding at least one of the frequency and the intensity of a magnetic field pulse by the parameter, and the step of generating the magnetic field pulse of the determined frequency and intensity.

According to still another aspect of the present disclosure, there is provided a method of stimulating a muscle using a magnetic field pulse, including the step of deciding a parameter based on at least one of the amount of muscle to be stimulated and the thickness of a fat layer outside the muscle of a user, the step of deciding at least one of the frequency and the intensity of a magnetic field pulse by the parameter, and the step of generating the magnetic field pulse of the determined frequency and intensity to stimulate the muscle.

In the muscle stimulation apparatus using a magnetic field pulse, the method of controlling the same, and the method of stimulating a muscle using the same according to the present disclosure, it is possible to decide the depth and the intensity of stimulation based on muscle mass and the thickness of a fat layer to perform muscle stimulation optimized for each individual and each muscle.

### BRIEF DESCRIPTION OF THE DRAWINGS

FIG. 1 is a perspective view of a muscle stimulation apparatus using a magnetic field pulse according to an embodiment of the present disclosure.
FIG. 2 is a view schematically showing an electrical circuit of the muscle stimulation apparatus using a magnetic field according to an embodiment of the present disclosure.
FIG. 3a, 3b and 3c are schematic views illustrating the depth of muscle stimulation that changes depending on the thickness of a fat layer.
FIG. 4 is a schematic view illustrating the concept of a magnetic field formed by an applicator.
FIG. 5a, 5b and 5c are showing frequencies of magnetic field pulses and depths of muscle stimulation.
FIGS. 6 to 8 are views illustrating a change in the frequency and the intensity of a magnetic field pulse for stimulating a muscle depending on the thickness of a fat layer.
FIG. 9 is a view illustrating the concept of a parameter update by electromyography.
FIG. 10 is a flowchart of a method of controlling the muscle stimulation apparatus using a magnetic field pulse according to another embodiment of the present disclosure.
FIG. 11 is a flowchart of a method of stimulating a muscle using a magnetic field pulse according to still another embodiment of the present disclosure.

### DESCRIPTION OF EXEMPLARY EMBODIMENTS

Hereinafter, a muscle stimulation apparatus using a magnetic field pulse, a method of controlling the same, and a method of stimulating a muscle using the same according to the embodiments of the present disclosure will be described in detail with reference to the appended drawings. In addition, the components in the description of the embodiments below may be called by different names in the industry. However, if the functions of the components are similar or the same, they can be deemed identical to each other even if a modified embodiment has been adopted. Furthermore, a reference sign of each component is given for the convenience of description. However, the content shown in the drawings where the signs are indicated does not limit each component to the scope of the content in the drawings. Similarly, even if an embodiment in which a feature in the drawings is partially modified is employed, the features can be deemed identical to each other when they are similar or the same in terms of function. In addition, when a certain component is considered as a component to be included as a matter of course in view of the level of knowledge of a persona having ordinary skill in the technical field, no description thereof will be provided.

The following description will be made on the premise that the term "muscle" in the description means a tissue that is contracted by stimulation such as muscles and sphincters included in a musculoskeletal system.

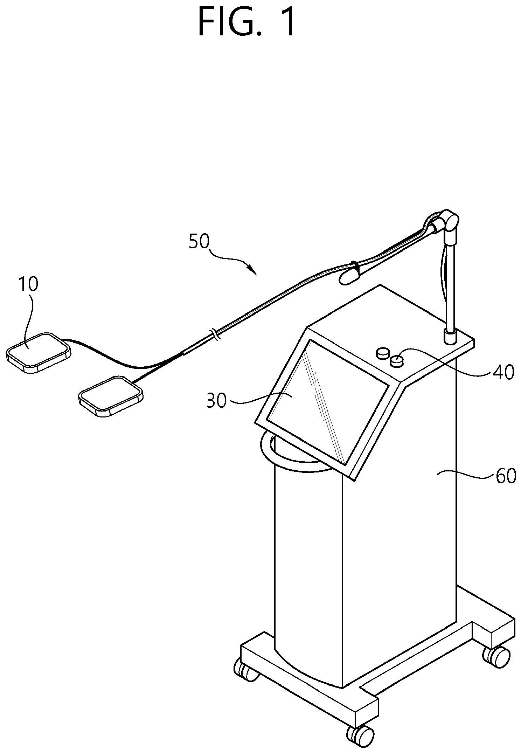

FIG. 1 is a perspective view of a muscle stimulation apparatus using a magnetic field pulse according to an embodiment of the present disclosure.

Referring to FIG. 1, the muscle stimulation apparatus using a magnetic field pulse according to an embodiment of the present disclosure may include a body portion 60, a power supply unit (not shown), an applicator 10, a display unit 30, an input unit 40, a controller (not shown), and a cable 50.

The body portion 60 may serve as a base on which other components are provided. There may be an empty space inside the body portion, and the power supply unit and the controller may be provided therein. In addition, although not shown, the body portion according to this embodiment of the present disclosure may include power sources and various electrical circuits such as an impedance matching circuit for operating the muscle stimulation apparatus. However, since these electrical circuits and power sources are widely used, detailed descriptions thereof will not be provided herein.

The power supply unit may supply power for generating magnetic field pulses. Furthermore, the power supply unit may supply power for electronic elements provided in the body portion and the applicator.

One side of the applicator 10 may be formed to be closely attached to a user's body, and the applicator 10 may receive power from the power supply unit and form a magnetic field. A coil may be provided as a magnetic field generator inside the applicator. The coil may be capable of generating a magnetic field pulse of a predetermined frequency under the control of the controller. The coil may also generate a magnetic field having an intensity determined by the control of the controller. A plurality of applicators may respectively generate a magnetic field pulse, and each magnetic field pulse may have a different frequency and intensity.

The plurality of applicators may be provided, and each applicator may stimulate a different muscle. The applicators may respectively generate a magnetic field pulse. Thus, the applicators may generate a specific movement in a body by respectively stimulating muscles related to each other, e.g., the muscles of brachial biceps and triceps, which contract and relax in opposite directions. For example, the applicators may be closely attached to the biceps and the triceps, respectively, and a magnetic field pulse for contracting a muscle may be alternately applied to the applicators to cause an operation where a muscle is alternately contracted and relaxed.

By the display unit 30 and the input unit 40, a user may monitor and control the apparatus for treatment. The user may control parameters related to treatment, such as treatment mode, treatment position, treatment intensity, and treatment time by the display unit 30 and the input unit 40. Meanwhile, the display unit 30 and the input unit 40 may be applied in various modified forms, e.g., a touchable display.

The controller (not shown) may control the overall operation of the muscle treatment apparatus. A user may input data to select a treatment mode, or a magnetic field pulse may be adjusted as the user inputs data to perform the operations of the start of treatment, an emergency stop in the middle of treatment, a change of a parameter in the middle of treatment, and a change of a treatment mode in the middle of treatment.

The controller (not shown) may decide a parameter based on muscle mass and the thickness of a fat layer surrounding a muscle. The operation of deciding the parameter may be performed to adjust the intensity and the depth of muscle stimulation by a magnetic field pulse. In addition, the operation of deciding the parameter may be performed to decide the frequency of an initial magnetic field pulse for contracting a muscle.

The controller may decide the on/off timing by a switch on an RC resonance circuit to generate a magnetic field pulse with specific frequencies. For example, the controller may perform on/off control of the switch to generate a magnetic field of a frequency with the range of 30 to 80 Hz. Furthermore, the controller may change the frequency of a magnetic field pulse during the operation of the apparatus, and may function to adjust the intensity of the magnetic field pulse.

The cable 50 may connect the body portion and the applicators. A plurality of cables may connect each applicator to the body portion.

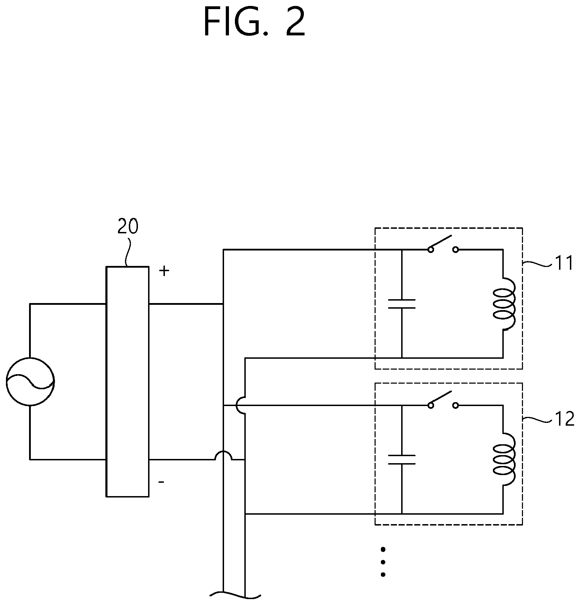

FIG. 2 is a view schematically showing an electrical circuit of the muscle stimulation apparatus using a magnetic field according to an embodiment of the present disclosure.

Referring to FIG. 2, the muscle stimulation apparatus using a magnetic field may include a circuit 20 for receiving power from the power supply unit and converting it into an appropriate characteristic. The power supply unit may have the capacity to simultaneously operate the plurality of applicators. The circuit 20 may include a circuit for converting alternating current into direct current. The circuit 20 may be connected in parallel to each applicator.

Meanwhile, the RC resonant circuit may include a capacitor and an inductor, which are well known, and the capacitor and the inductor may be modified so that a capacitance value and an inductance value are adjusted. When power is supplied by the power supply unit, electric charges may be stored in the capacitor, and each applicator may generate a magnetic field pulse at last while resonating with the inductor by the operation of the switch.

Each RC resonant circuit may be partially or fully provided to each applicator, and a magnetic field pulse may be formed by the inductor or the coil. For example, a first applicator 11 of the plurality of applicators may include both the capacitor and the inductor, and a second applicator 12 may include the inductor. In this case, the capacitor connected to the second applicator 12 may be provided in the body portion.

The plurality of applicators may respectively include the switch that can be independently actuated. The switch may determine whether to operate the RC resonant circuit. Each switch may be controlled by the controller.

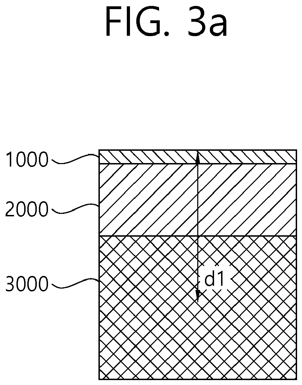

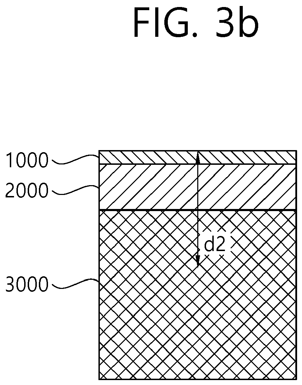

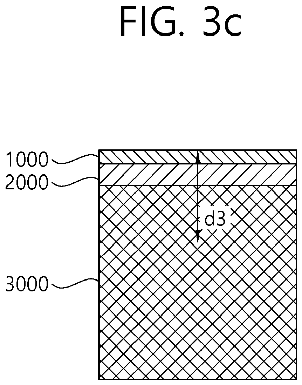

FIG. 3a, 3b and 3c are schematic views illustrating the depth of muscle stimulation that changes depending on the thickness of a fat layer.

Referring to FIG. 3a, 3b and 3c, in general, epidermis 1000 may not vary significantly between individuals or body parts. On the other hand, a fat layer 2000 and a muscle 3000 may vary significantly between individuals or body parts. In the case of a small muscle mass, when excessively strong stimulation is transmitted, pain may occur and the effect of muscle stimulation may be reduced. In addition, in the case of a large muscle mass, when weak stimulation is transmitted, an appropriate level of muscle contraction may not be caused. Therefore, it may be desirable to determine a target stimulation depth for muscle stimulation and the intensity of a magnetic field pulse in order to stimulate a muscle at an appropriate level for each individual and body part.

FIG. 3a shows an example of the fat layer 2000 thick such as that of a woman, an obese person, etc. In this case, the target stimulation depth needs to be increased. In contrast, FIG. 3c shows an example of the fat layer 2000 thin such as that of a man who works out a lot, etc. In this case, it may be possible to stimulate the muscle 3000 even when a penetration depth is shallow, so the target stimulation depth is shallow. On the other hand, the target stimulation depth in FIG. 3b may be set to be between the target stimulation depth in FIG. 3a and the target stimulation depth in FIG. 3c. Meanwhile, the intensity of a magnetic field pulse may be adjusted based on muscle mass. For example, as shown in FIG. 3c, in the case of the muscle 3000 relatively thick such as that of a bodybuilder, an athlete, etc., the intensity of a magnetic field pulse may be increased to generate a strong contraction. On the other hand, when muscle mass is small as shown in FIG. 3a, sufficient contraction may occur even when the intensity of a magnetic field pulse is relatively weak.

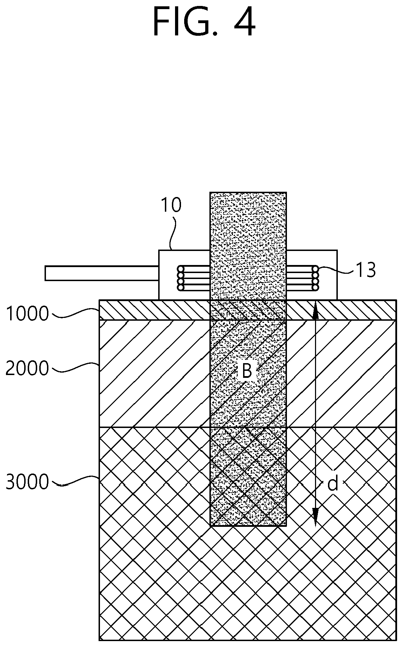

FIG. 4 is a schematic view illustrating the concept of a magnetic field formed by the applicator.

FIG. 4 shows a state where the magnetic field pulse formed by any one of the applicators 10 according to this embodiment has sequentially passed through the epidermis 1000 and the fat layer 2000 and then reached the inside of the muscle 3000. As described above, when a current is applied to the applicator, the strongest area of a magnetic field B may be formed in the center of the coil 13, which is the magnetic field generator. An area where the density of a magnetic field is low is not shown in the drawings for the convenience of description. The distance to the bottom of an area having a certain level of the density of a magnetic field may be the target stimulation depth d. As a magnetic field pulse is formed, an induced current may be generated within a muscle, and the muscle may be stimulated by the induced current. In this case, it may be possible to adjust the target stimulation depth by adjusting the frequency of the magnetic field pulse as described above.

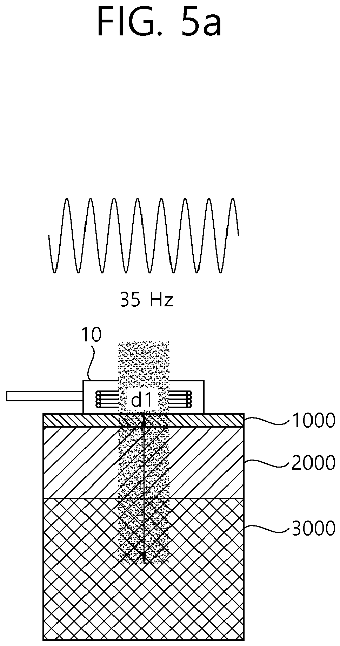

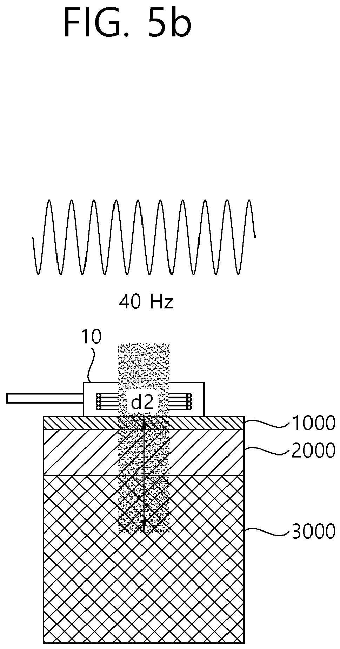

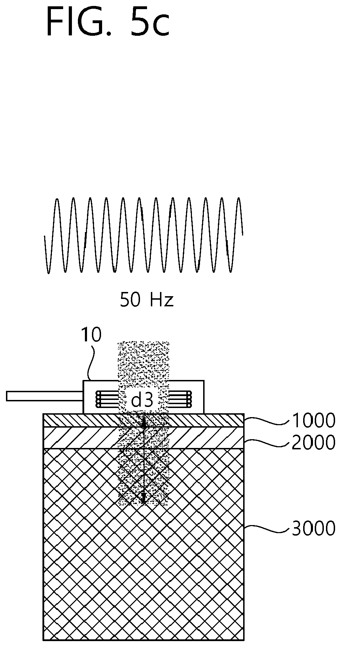

FIG. 5a, 5b and 5c are showing frequencies of magnetic field pulses and depths of muscle stimulation.

It is known that the depth of penetration of a magnetic field pulse decreases as frequency increases within a low frequency range of 10 to 100 Hz.

Referring to FIG. 5a, when the frequency of a magnetic field pulse is 35 Hz, a muscle may be stimulated to a first target stimulation depth d1, and, when the frequency of the magnetic field pulse is 40 Hz as shown in FIG. 5b, the muscle may be stimulated to a second target stimulation depth d2 that is shallower than the first target stimulation depth d1. In addition, when the frequency of the magnetic field pulse is 50 Hz as shown in FIG. 5c, the muscle may be stimulated under the influence of the magnetic field to a third target stimulation depth d3, which is the shallowest depth.

The controller may decide a parameter based on muscle mass and the thickness of a fat layer. The parameter may affect the decision on the frequency and the intensity of a magnetic field pulse. Meanwhile, a user may decide his/her muscle mass and the thickness of his/her fat layer based on the results of tomography, an analysis of body composition based on impedance, etc. The controller may decide a parameter to be set based on information on the muscle mass and the thickness of the fat layer.

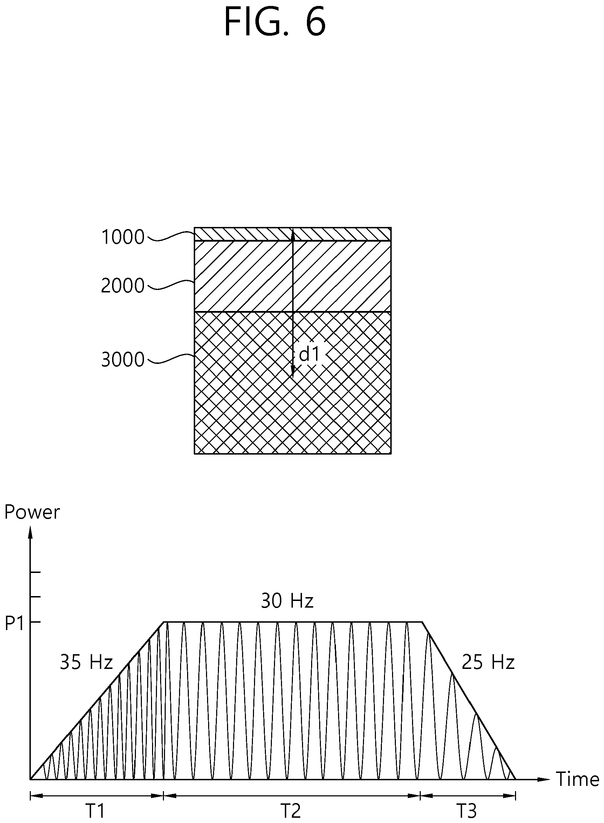

The decision on the parameter by the controller, a change of the frequency of a magnetic field pulse, and the control of the intensity of the magnetic field pulse will be described in detail with reference to FIGS. 6 to 8 below.

FIGS. 6 to 8 are views illustrating a change in the frequency and the intensity of a magnetic field pulse for stimulating a muscle depending on the thickness of a fat layer.

In the sequence in which a magnetic field pulse is generated, the controller may decide the target stimulation depth d1, d2, and d3 based on information on muscle mass and the thickness of a fat layer for the magnetic field pulse to pass through the fat layer 2000 from the epidermis 1000 and then reach the inside of the muscle 3000, and may decide the intensity of muscle stimulation. Thereafter, to initiate the sequence in which a magnetic field pulse is generated, the controller may adjust the parameter, set a first frequency, a second frequency, and a third frequency, and set the intensity of the magnetic field pulse.

Muscle tension may be subject to the interval between electric currents generated by a magnetic field pulse. Therefore, during the first muscle contraction, a magnetic field pulse of the first frequency, which is the highest frequency, may be generated. When a muscle is contracted by one magnetic field pulse before part of the muscle is relaxed, the next magnetic field pulse may be applied so that the muscle may be contracted again. When such a stimulation is repeatedly delivered, the level of the contraction of the muscle may rise. Meanwhile, a magnetic field pulse of the second frequency lower than the first frequency may be generated in order to maintain the contraction of the muscle. At this point, it is sought to maintain the contraction rather than to raise the level of the contraction of the muscle. Accordingly, the frequency of the magnetic field pulse may be slightly lower than that of the initial contraction to prevent excessive muscle contraction, but the intensity of the magnetic field pulse may be maintained strong to maintain the overall muscle contraction. On the other hand, when the muscle is relaxed, the frequency may be further lowered so that the entire muscle may be relaxed with a contraction generated at a regular interval, and the intensity of the magnetic field pulse may be gradually decreased before the magnetic field pulse may disappear in the end to create a rest period of the muscle.

The controller may adjust a value of the parameter so that the target stimulation depth is deeper as the amount of fat is greater, and may also adjust the parameter value so that the target stimulation depth is shallower as the amount of fat is less.

FIG. 6 shows the concept of stimulating the muscle shown in figure (a) of FIG. 3 described above.

The controller may operate in the sequence in which a muscle is stimulated based on stored information on muscle mass and the thickness of a fat layer. In this case, the controller may set the first target stimulation depth d1 based on the information on the muscle mass and the thickness of the fat layer, and may set a parameter for a magnetic field pulse to go down to the target stimulation depth d1. For example, the parameter may set the first frequency for generating the first contraction of muscle. FIG. 6 shows an example where the first frequency is set to 35 Hz by the parameter, corresponding to the first target stimulation depth.

During each operating time, the sequence in which a magnetic field pulse for muscle stimulation is generated may be divided into a first section, a second section, and a third section.

The first section may be a section where a muscle is stimulated and contraction of the muscle is caused. In the first section, the controller may generate a magnetic field pulse of the first frequency set by the parameter and gradually increase the intensity of the magnetic field pulse up to a first intensity P1.

The second section may correspond to the step of maintaining the contracted muscle. In this step, when an appropriate level of stimulation is delivered to the muscle, the stimulation of the muscle and the destruction of the muscle fiber may be actually generated, and then the muscle may be strengthened as the body recovers. The controller may maintain the intensity of the magnetic field pulse at the first intensity P1 in the second section. Meanwhile, in the second section, the controller may change the frequency of the magnetic field pulse to the second frequency, e.g., 30Hz, which is lower than the first frequency, in order to maintain the contracted muscle.

The third section may correspond to the step of transition for relaxing the contracted muscle. The controller may stop generating the magnetic field by gradually lowering the intensity of the magnetic field pulse in the third section. In addition, in the third section, the controller may change the frequency of the magnetic field pulse to the third frequency, e.g., 25Hz, which is lower than the second frequency.

Meanwhile, the controller may control the sequence in which stimulation of the entire muscle occurs to be repeated several times or dozens of times during a preset operating time.

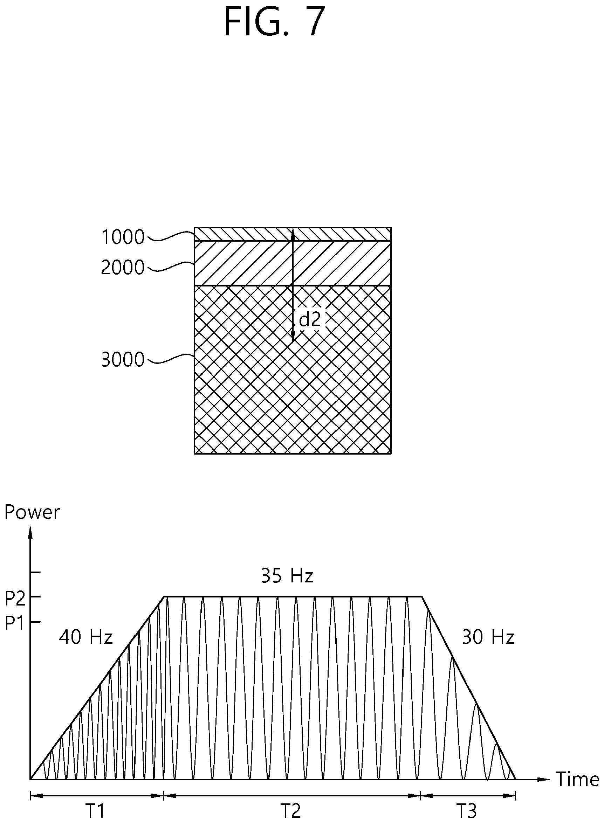

FIG. 7 shows the concept of stimulating the muscle shown in figure (b) of FIG. 3 described above. As described with reference to FIG. 6, the controller may change the frequency of a magnetic field pulse by setting a parameter. The controller may set the parameter to set the first frequency corresponding to the second target stimulation depth d2. Thereafter, the controller may control the sequence in which a magnetic field pulse is generated to be divided into the first section to the third section so that the frequency for generating the magnetic field pulse in each section may be gradually lowered. In addition, the controller may decide the intensity of the magnetic field pulse generated in the second section.

For example, the controller may set the first frequency of the magnetic field pulse to 40 Hz, the second frequency to 35 Hz, and the third frequency to 30 Hz. In addition, in the second section, the controller may set the intensity of the magnetic field pulse to a second intensity P2 stronger than the intensity shown in FIG. 6, which is to apply an appropriate level of stimulation to a muscle greater than the muscle shown in FIG. 6.

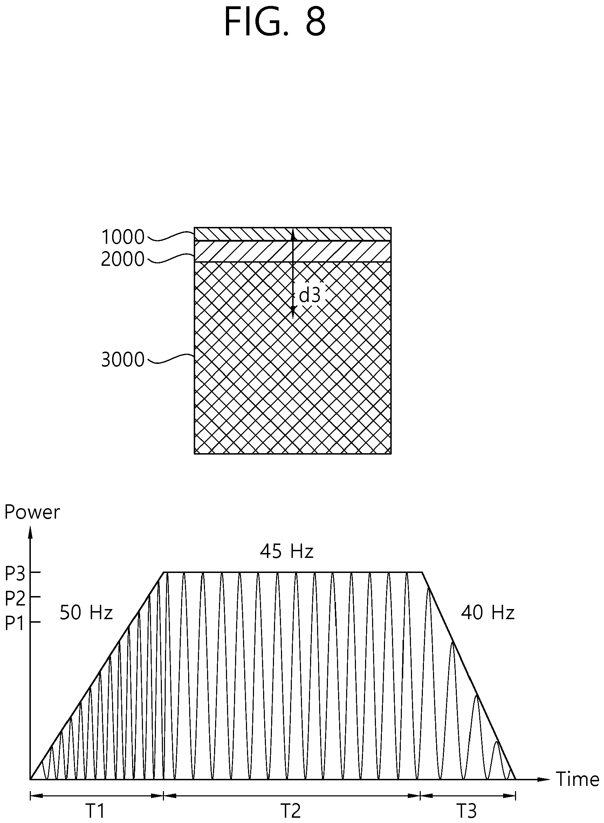

FIG. 8 shows the concept of stimulating the muscle shown in figure (c) of FIG. 3 described above. Since the tissue shown in the figure has a thin thickness of a fat layer and a large amount of muscle, the controller may set the target stimulation depth d3 to be shallow and may set the first frequency, which is an initial frequency, to 50 Hz. Thereafter, the controller may change the frequency of the magnetic field pulse to 45 Hz in the second section and to 40 Hz in the third section. Meanwhile, the intensity of the magnetic field pulse in the second section may be set to a third intensity P3 that is stronger than the intensity in FIGS. 6 and 7.

That is, it may possible that the controller generates a sequence to create an appropriate magnetic field pulse by adjusting the parameter and generates an optimized magnetic field pulse based on muscle mass and the thickness of a fat layer of a user to generate an appropriate level of stimulation for the muscle.

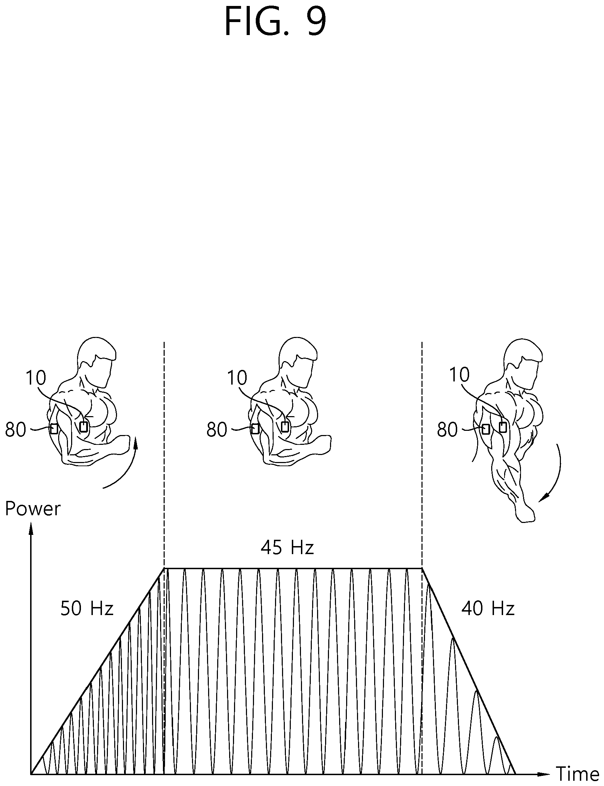

FIG. 9 is a view illustrating the concept of a parameter update by electromyography (EMG).

The muscle stimulation apparatus using a magnetic field pulse according to an embodiment of the present disclosure may include a sensor. The sensor may be provided to perform the EMG.

Signal measured by the EMG sensor 80 may be transformed into frequencies by Fourier transform to indicate the level of muscle fatigue. As an example, a Fourier transform may be performed on an EMG signal, a spectrum evaluation method may be used, and muscle fatigue may be quantitatively evaluated by comparing a ratio of spectral density within a specific frequency range. The EMG sensor 80 may be attached to a muscle related to a muscle that is being currently contracted by the applicator 10 for the EMG. For example, when a biceps muscle is stimulated by the applicator, the EMG sensor 80 may be attached to a triceps muscle for the measurement of the level of muscle fatigue. The fatigue of a contiguous muscle may also be measured when measuring the fatigue of a muscle so that the fatigue of the muscle that is actually stimulated may be calculated. In this case, the measurement by the EMG sensor 80 may be made while a magnetic field pulse is not applied after each operating time has been completed. That is, during the rest period when no magnetic field pulse is applied after each round of the operation for muscle contraction, maintenance of muscle contraction, and muscle relaxation, the EMG sensor 80 may measure the level of muscle fatigue.

When the measurement of muscle fatigue is completed based on a value measured by the EMG sensor 80, the controller may control the sequence for generating a magnetic field pulse based on the measured muscle fatigue. In other words, when a high muscle fatigue is measured, the controller may prematurely end the sequence for generating a magnetic field pulse. In addition, when the muscle fatigue is not increased to a high level even after the sequence for generating a magnetic field pulse has been performed for a predetermined time, the controller may increase the intensity of the magnetic field pulse so that the muscle may be more strongly stimulated.

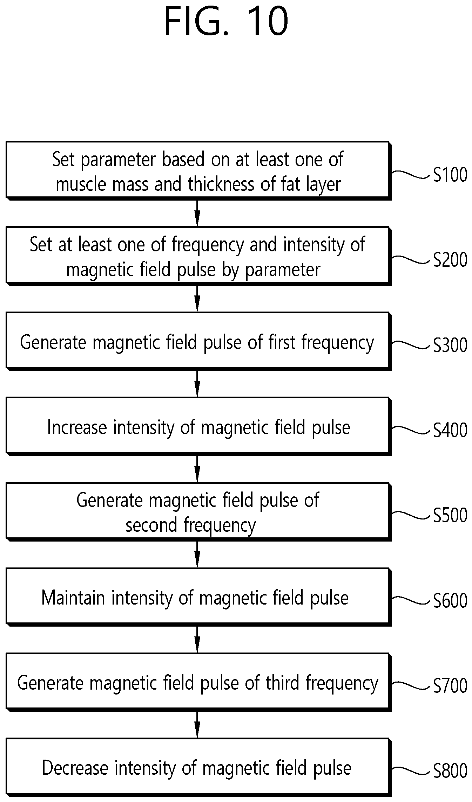

Hereinafter, a method of controlling the muscle stimulation apparatus using a magnetic field pulse according to another embodiment of the present disclosure will be described with reference to FIG. 10.

FIG. 10 is a flowchart of the method of controlling a muscle stimulation apparatus using a magnetic field pulse according to another embodiment of the present disclosure.

Referring to FIG. 10, the method of controlling the muscle stimulation apparatus using a magnetic field pulse according to another embodiment of the present disclosure may include the step of setting a parameter based on at least one of muscle mass and the thickness of a fat layer at S100, the step of setting at least one of the frequency and the intensity of a magnetic field pulse by the parameter at S200, the step of generating a magnetic field pulse of a first frequency at S300, the step of increasing the intensity of the magnetic field pulse at S400, the step of generating a magnetic field pulse of a second frequency at S500, the step of maintaining the intensity of the magnetic field pulse at S600, the step of generating a magnetic field pulse of a third frequency at S700, and the step of decreasing the intensity of the magnetic field pulse at S800.

In the step of setting a parameter based on at least one of muscle mass and the thickness of a fat layer at S100, the parameter may be set based on information on a muscle to be stimulated and the thickness of a fat layer outside the muscle. In this step, the target stimulation depth of a magnetic field pulse may be determined based on the thickness of the fat layer and the muscle mass, and the parameter for generating a predetermined level of magnetic field that goes down to the target stimulation depth may be set.

In the step of setting at least one of the frequency and the intensity of a magnetic field pulse by the parameter at S200, at least one of the frequency and the intensity of the magnetic field pulse may be set based on a value of the parameter corresponding to the target stimulation depth. In this step, the frequency of the magnetic field pulse may be set to be lower as the target stimulation depth is increased, whereas the frequency of the magnetic field pulse may be set to be higher as the target stimulation depth is decreased. In addition, the intensity of the magnetic field pulse may be set in proportion to muscle mass. In other words, the intensity of the magnetic field pulse may be set to be stronger as the muscle mass increases, whereas the intensity of the magnetic field pulse may be set to be weaker as the muscle mass decreases.

In the step of generating a magnetic field pulse of a first frequency at S300, the magnetic field pulse of the first frequency set by the parameter may be generated. The magnetic field pulse generated in this step may stimulate a muscle by generating an induced current within the muscle.

In the step of increasing the intensity of the magnetic field pulse at S400, the magnetic field pulse of the first frequency may be applied, but the intensity of the magnetic field pulse may be gradually increased. The magnetic field pulse generated in this step may cause muscle contraction.

In the step of generating a magnetic field pulse of a second frequency at S500, the frequency of the magnetic field pulse may be changed to the second frequency lower than the first frequency.

In the step of maintaining the intensity of the magnetic field pulse at S600, the magnetic field pulse whose frequency has been changed to the second frequency may be applied, but the intensity of the magnetic field pulse may be maintained for a predetermined time. The muscle contraction may be maintained by the magnetic field generated in this step.

In the step of generating a magnetic field pulse of a third frequency at S700, the frequency of the magnetic field pulse may be changed to the third frequency lower than the second frequency.

In the step of decreasing the intensity of the magnetic field pulse at S800, the frequency of the magnetic field pulse may be changed to the third frequency, but the intensity of the magnetic field pulse may be gradually decreased. The magnetic field generated in this stage may gradually disappear, and an electric current that stimulates the muscle may also disappear in the end, so that the muscle may naturally relax.

Meanwhile, this step (S800) may take longer to be completed than the step (S400) of increasing the intensity of the magnetic field pulse because the level of muscle stimulation tends to increase when a user makes a motion for relaxing a muscle more slowly than when making a motion for contracting a muscle while actually working out.

In the meantime, the method of controlling a muscle stimulation apparatus using a magnetic field pulse described above may be applied to the muscle stimulation apparatus using a magnetic field pulse described with reference to FIG. 1. In addition, the method of controlling a muscle stimulation apparatus using a magnetic field pulse according to this embodiment of the present disclosure may be applied to each of the plurality of applicators provided in the muscle stimulation apparatus.

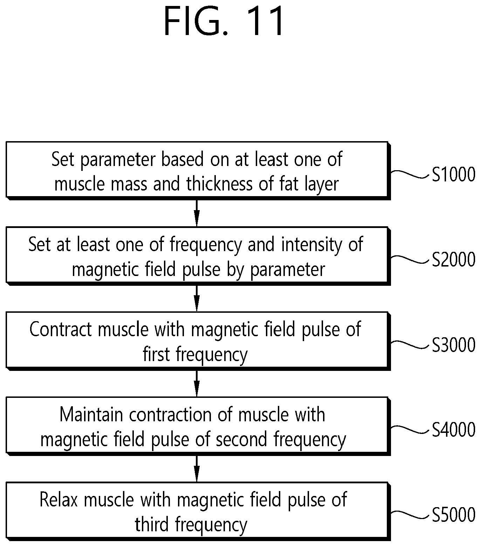

Hereinafter, a method of stimulating a muscle using a magnetic field pulse according to still another embodiment of the present disclosure will be described with reference to FIG. 11.

FIG. 11 is a flowchart of the method of stimulating a muscle using a magnetic field pulse according to still another embodiment of the present disclosure.

Referring to FIG. 11, the method of stimulating a muscle using a magnetic field pulse according to still another embodiment of the present disclosure may include the step of setting a parameter based on at least one of muscle mass and the thickness of a fat layer at S1000, the step of setting at least one of the frequency and the intensity of a magnetic field pulse by the parameter at S2000, the step of contracting the muscle with a magnetic field pulse of a first frequency at S3000, the step of maintaining the contraction of the muscle with a magnetic field pulse of a second frequency at S4000, and the step of relaxing the muscle with a magnetic field pulse of a third frequency at S5000.

In the step of setting a parameter based on at least one of muscle mass and the thickness of a fat layer at S1000, the amount of muscle to be stimulated and the thickness of the fat layer outside the muscle may be measured. The measurement of the muscle mass and the thickness of the fat layer may be performed by tomography, an analysis of body fat based on impedance, etc. before the muscle is stimulated. In this step, a target stimulation depth for a magnetic field pulse to reach the muscle layer through the fat layer may be set based on the muscle mass and the thickness of the fat layer that have been measured. When the target stimulation depth has been set, the parameter may be set. In this step, information on gender, age, muscle mass and fat mass, and the thickness of a fat layer may be referred to in order to set a parameter. Meanwhile, in this step, a plurality of parameters may be set.

In the step of setting at least one of the frequency and the intensity of a magnetic field pulse by the parameter at S2000, the frequency and the intensity of the magnetic field pulse generated based on the parameter set according to the target stimulation depth and the muscle mass may be set. The process in this step may be performed before the magnetic field pulse is generated. In addition, in this step, the initial frequency and intensity of the magnetic field pulse may be set, and the frequency and the intensity of a magnetic field pulse to be generated in the next section may be set while the magnetic field pulse is applied. That is, the frequency and the intensity of a magnetic field pulse may be preset or may be set in real time when the muscle is stimulated.

In the step of contracting the muscle with a magnetic field pulse of a first frequency at S3000, the first frequency, which is the highest frequency of each round of the treatment process, may be applied to contract the muscle. In this step, the magnetic field pulse of the first frequency may be applied, but the intensity of the magnetic field pulse may be gradually increased during a first period of time. In this case, the final intensity of the magnetic field pulse may be preset by the parameter.

In the step of maintaining the contraction of the muscle with a magnetic field pulse of a second frequency at S4000, the magnetic field pulse having the second frequency lower than the first frequency may be delivered to the muscle. When the magnetic field pulse of the second frequency is applied to the muscle, the intensity of the magnetic field pulse may be maintained so that the contracted muscle may be maintained while the process of this step is performed.

In the step of relaxing the muscle with a magnetic field pulse of a third frequency at S5000, the frequency of the magnetic field pulse may be changed to the third frequency lower than the second frequency, and the intensity of the magnetic field pulse may be gradually adjusted to a weaker level, thereby relaxing the muscle. This step (S5000) may take longer to be completed than the step (S3000) of contracting the muscle.

As described above, in the muscle stimulation apparatus using a magnetic field pulse, the method of controlling the same, and the method of stimulating a muscle using the same according to the present disclosure, it may be possible to set the depth and the intensity of stimulation based on muscle mass and the thickness of a fat layer. Accordingly, it may be possible to perform muscle stimulation optimized for each individual and each muscle while reducing pain and minimizing any possible inconvenience in using the apparatus.

## Claims

1. A muscle stimulation apparatus using a magnetic field pulse, comprising:
a power supply unit;
an applicator (10) including a magnetic field generator (13) capable of generating a magnetic field pulse by receiving power from the power supply unit; and
a controller capable of setting at least one of the frequency and the intensity of a magnetic field pulse generated by the magnetic field generator,
**characterized in that** the controller adjusts a parameter for setting at least one of the frequency and the intensity of the magnetic field pulse in the middle of performance of a preset treatment process, and
a value of the parameter is determined based on at least one of the amount of muscle to be stimulated and the thickness of a fat layer outside the muscle of a user.

2. The muscle stimulation apparatus of claim 1,
wherein the controller adjusts a value of the parameter to adjust a target stimulation depth for the magnetic field to reach the inside of the muscle from the user's epidermis based on the muscle mass and the thickness of the fat layer of the user.

3. The muscle stimulation apparatus of claim 2,
wherein the controller adjusts a value of the parameter so that the target stimulation depth is deeper as the amount of the fat is greater.

4. The muscle stimulation apparatus of claim 2,
wherein the controller adjusts a value of the parameter so that the target stimulation depth is shallower as the amount of the fat is less.

5. The muscle stimulation apparatus of claim 1,
wherein the controller controls the magnetic field generator to adjust the intensity and the frequency of a magnetic field generated during a preset operating time at least twice.

6. The muscle stimulation apparatus of claim 5,
wherein the controller sets a first frequency that causes muscle contraction by stimulating the muscle based on the parameter, and controls the magnetic field generator based on the first frequency.

7. The muscle stimulation apparatus of claim 6,
wherein the controller generates the magnetic field pulse of the first frequency during a first section, which is an initial section of the operating time, and controls the magnetic field generator to gradually increase the intensity of the magnetic field pulse to a level that causes muscle contraction.

8. The muscle stimulation apparatus of claim 7,
wherein the controller generates the magnetic field pulse of a second frequency lower than the first frequency during a second section after the first section of the operating time, and controls the magnetic field generator to maintain the intensity of the magnetic field pulse at the intensity that causes muscle contraction.

9. The muscle stimulation apparatus of claim 8,
wherein the controller generates the magnetic field pulse of a third frequency lower than the second frequency during a third section after the second section of the operating time, and controls the magnetic field generator to gradually decrease the intensity of the magnetic field pulse.

10. The muscle stimulation apparatus of claim 9,
wherein the first frequency is in the range of 30 to 60 Hz.

11. The muscle stimulation apparatus of claim 9,
wherein the controller adjusts the parameter to set the first frequency to 35 Hz, the second frequency to 40 Hz, and the third frequency to 45 Hz.

12. The muscle stimulation apparatus of claim 2, further comprising
at least one sensor for electromyography,
wherein the controller adjusts the parameter based on a value measured by the sensor.

13. The muscle stimulation apparatus of claim 12,
wherein the controller receives at least one signal from the sensor when the magnetic field pulse is not applied to the muscle.

14. The muscle stimulation apparatus of claim 13,
wherein the controller adjusts the parameter to adjust the intensity of the magnetic field pulse stronger when the controller determines that the muscle fatigue is low by analyzing the signal.

15. The muscle stimulation apparatus of claim 13,
wherein the controller adjusts the parameter to adjust the intensity of the magnetic field pulse weaker or cuts off the magnetic field when the controller determines that the muscle fatigue is high by analyzing the signal.
